(19) Europäisches Patentamt
European Patent Office
Office européen des brevets

(11) Publication number: 0 253 517 B1

(12) EUROPEAN PATENT SPECIFICATION

(45) Date of publication of patent specification: 25.03.92 (51) Int. Cl.5: A61K 31/70

(21) Application number: 87305410.0

(22) Date of filing: 18.06.87

# (54) Use of 3'azido-3'deoxythymidine for the manufacture of a medicament for the treatment of giardiasis.

(30) Priority: 19.06.86 US 876234

(43) Date of publication of application: 20.01.88 Bulletin 88/03

(45) Publication of the grant of the patent: 25.03.92 Bulletin 92/13

(84) Designated Contracting States: BE CH DE FR GB IT LI NL SE

(73) Proprietor: THE WELLCOME FOUNDATION LIMITED
Unicorn House 160 Euston Road
London NW1 2BP(GB)

(72) Inventor: Miller, Richard Lee
4100 Picardy Drive
Raleigh, NC 27612(US)

(74) Representative: Garrett, Michael et al
The Wellcome Research Laboratories Group
Patents and Agreements Langley Court
Beckenham Kent BR3 3BS(GB)

EP 0 253 517 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the treatment of giardiasis with 3′-azido-3′-deoxythymidine or its pharmaceutically acceptable esters or basic salts.

Giardiasis in human beings is caused by Giardia lamblia (also called Giardia intestinalis), flagellate protozoan which is found in almost all parts of the world. This parasite, considered to be the leading protozoan cause of diarrhoea among travellers, is predominently water-borne, but person-to-person and food-borne transmission can also occur. Internationally it is one of the major causes of diarrhoeal disease in infants and young children and thus contributes significantly to the excessive morbidity and mortality in children of developing countries.

Domestic and certain wild animals are also susceptible to giardiasis. G. canis is the species infecting dogs, although crossover infections have been reported between human beings and dog and between beaver and human beings.

We have now found that 3′-azido-3′-deoxythymidine (which has the approved name zidovudine) is active against Giardia lamblia and is therefore useful for the treatment or prophylaxis of giardiasis.

According to the present invention we provide the use of 3′-azido-3′-deoxythymidine, i.e. the compound of formula (I):-

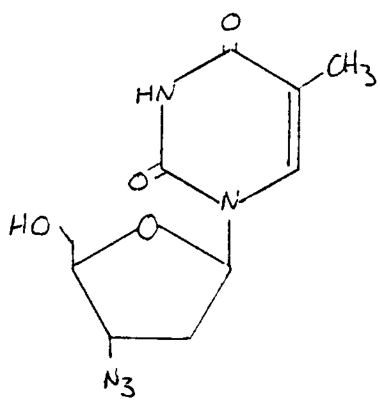

(I)

or a pharmaceutically acceptable salt or ester thereof in the manufacture of a pharmaceutical composition for the treatment or prophylaxis of a giardial infection in an animal.

As indicated above, giardiasis occurs in non-human animals particularly mammals although the invention is especially applicable to the treatment or prophylaxis of giardiasis in humans.

Pharmaceutically acceptable salts which are especially preferred for therapeutic use are salts of alkali metals such as sodium and potassium, alkaline earth metals such as calcium and magnesium and ammonium salts such as tetralkylammonium salts in which the alkyl groups may be straight or branched chain and may contain 1-5 carbon atoms preferably methyl or ethyl.

Preferred esters of the compound of formula (I) include carboxylic acid esters in which the non-carbonyl moiety of the ester grouping is selected from straight or branched chain alkyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); and mono-, di- or triphosphate esters. Any reference to any of the above compounds also includes a reference to a pharmaceutically acceptable salt thereof.

With regard to the above-described esters, unless otherwise specified, any alkyl moiety present advantageously contains 1 to 18 carbon atoms, particularly 1 to 4 carbon atoms. Any aryl moiety present in such esters advantageously comprises a phenyl group.

The salts of the compound of formula (I) are converted to the parent compound after administeration and are thus prodrugs. The compound 3′-azido-3′-deoxythymidine is believed to penetrate into the Giardia lamblia cells after contacting same and be converted in such cells by the giardial enzymes to the monophosphate thereof. The monophosphate is believed to be then convered by the giardial enzymes to the diphosphate of the compound of formula (I) and ultimately to the triphosphate of the compound of formula (I). Thus, the compound of formula (I) can be used to be a prodrug after entering the lamblia cells since it is an intermediate (precursor) to the mono-, di-, and triphosphate thereof.

All prodrugs (precursors) are administered to a human in an amount sufficient to generate an effective amount of the compound which contacts the Giardia lamblia and interacts with it (e.g., prevents replication

thereof).

The above mentioned pharmaceutically acceptable salts and esters may be prepared in a conventional manner, e.g., salts may be prepared by treatment of the compound of formula (I) with an appropriate base while the esters may be prepared for example by treatment of the compound of formula (I) with an appropriate esterifying agent e.g. an acyl halide or anhydride.

The compound of formula (I) has been disclosed for example in European Patent Specifications EP-A-196185 and EP-A-199451 as having activity against retroviruses such as a HTLV-III and Gram negative bacteria. The compound of formula (I) may be prepared in conventional manner, for example as described by Horwitz, et al., J. Org. Chem. 29 (1964) 2076. The above-mentioned European Patent Specifications also discloses further methods for the preparation of the compound of formula (I) and its pharmaceutically acceptable salts and esters.

The pharmaceutical compositions of this invention, for the treatment of giardial infections, may be given orally, topically in the mouth (e.g., buccal or sublingual), parenterally, as a suppository or by inhalation as an aerosol. The compositions are administered at dose levels of the compound of formula (I) or a pharmaceutically acceptable salt or ester (hereinafter thereof referred to as active ingredients), calculated as the free base, of 0.1 to 100mg per kg, preferably about 1 to about 40mg per kg, and most preferably about 2 to about 20mg per kg of mammal body weight per day, and are used in human beings in unit dosage form, administered 1 to about 4 times per day in an amount of about 2 to about 250 mg per unit dose.

While it is possible for the active ingredients to be administered alone it is preferable to present them as pharmaceutical formulations. The formulations, both for veterinary and for human use, of the present invention comprise at least one active ingredient, as above defined, together with one or more pharmaceutical carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical in the mouth (e.g., buccal or sublingual), or parenteral (including subcutaneous, intramuscular, intravenous and intradermal) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredients with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredients; as a powder or granules; as a solution or a suspension in an aqueous liquid or a non-aqueous liquid or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier. Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 µm which is administered in the manner in which snuff is taken, i.e., by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for patenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried

(lyophilized) condition requiring only the addition of the sterile liquid carrier, for example water for injections, immediately prior to use. Extemporaneous injection solutions and suspensions may be prepared from sterile powders, granules and tablets of the kind previously described. Preferred unit doseage formulations are those containing a daily dose or unit daily sub-dose, as herein above recited, or an appropriate fraction thereof, of an active ingredient. For parenteral administration the compound of formula (I) or a pharmaceutically acceptable salt or ester thereof may be presented in aqueous solution in a concentration of from 0.1 to 10%, preferably 0.1 to 5%, most preferably 0.2% w/v.

It should be understood that in addition to the ingredients particularly mentioned above the formulations of this invention may include other agents conventional in the art having regard to the type of formulation in question, for example those suitable for oral administration may include flavouring agents.

The present invention further provides veterinary compositions comprising the active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by other desired routes.

For oral administration the compositions can be in the form of a tablet, granule, drench, paste, cachet, capsule or feed supplement. Granules may be made by the well known techniques of wet granulation, precompression or slugging. They can be administered to animals in an inert liquid vehicle so as to form a drench, or in a suspension with water or an oil base. Preferably further accessory ingredients such as a dispensing agent are included. These formulations preferably contain from 15 to 85% of the active ingredient.

A paste may be formulated by suspending the active ingredient in a liquid diluent. A stiffening or thickening agent may be included together with a wetting agent or a humectant if the liquid diluent is water. If an emulsion paste is needed then one or more surface active agents should desirably be included. From 25 to 80% by weight of these paste formulations may comprise the active ingredient.

In feed supplements the active ingredient is generally present in large amounts relative to the accessory ingredients, and the supplements may be added directly or after intermediate blending or dilution. Examples of accessory ingredients for such formulations include solid, orally ingestible carriers such as corn meal, soya flour, wheat-shorts, soya grits, edible vegetable materials and fermentation residues. The active ingredient is usually incorporated in one or more of the accessory ingredients and intimately and uniformly dispersed by grinding, tumbling or stirring with conventional apparatus. Formulations containing 1 to 90% by weight of the active ingredient are suitable for adding to feeds.

The following Examples illustrate the present invention in which the "active ingredient" is the compound of formula (I)

Example 1: Tablet Formulations

The following formulations A and B are prepared by wet granulation of the ingredients with a solution of povidone, followed by addition of magnesium stearate and compression.

| Formulation A | | |
|---|---|---|
| | mg/tablet | mg/tablet |
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

| Formulation B | | |
|---|---|---|
| | mg/tablet | mg/tablet |
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

| Formulation C. | |
|---|---|
| | mg/tablet |
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose used in formulation E is of the direct compression type (Dairy Crest - "Zeparox").

| Formulation D | |
|---|---|
| | mg/capsule |
| Active Ingredient | 250 |
| Pregelatinised Starch NF15 | 150 |
| | 400 |

| Formulation E | |
|---|---|
| | mg/capsule |
| Active Ingredient | 250 |
| Lactose | 150 |
| Avicel | 100 |
| | 500 |

Formulation F (Controlled Release Formulation)

The formulation is prepared by wet granulation of the ingredients (below) with a solution of povidone followed by the addition of magnesium stearate and compression.

| | mg/tablet |
|---|---|
| (a) Active Ingredient | 500 |
| (b) Hydroxypropylmethylcellulose (Methocel K4M Premium) | 112 |
| (c) Lactose B.P. | 53 |
| (d) Povidone B.P.C. | 28 |
| (e) Magnesium Stearate | 7 |
| | 700 |

Example 2: Capsule Formulations

Formulation A

A capsule formulation is prepared by admixing the ingredients of Formulation D in Example 1 above and filling into a two-part hard gelatin capsule. Formulation B (infra) is prepared in a similar manner.

| Formulation B | |
|---|---|
| | mg/capsule |
| (a) Active ingredient | 250 |
| (b) Lactose B.P. | 143 |
| (c) Sodium Starch Glycollate | 25 |
| (d) Magnesium Stearate | 2 |
| | 420 |

| Formulation C | |
|---|---|
| | mg/capsule |
| (a) Active ingredient | 250 |
| (b) Macrogol 4000 BP | 350 |
| | 600 |

Capsules are prepared by melting the Macrogol 4000 BP, dispersing the active ingredient in the melt and filling the melt into a two-part hard gelatin capsule.

| Formulation D | |
|---|---|
| | mg/capsule |
| Active ingredient | 250 |
| Lecithin | 100 |
| Arachis Oil | 100 |
| | 450 |

Capsules are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients a, b and c using an extruder, followed by spheronisation of the extrudate and drying. The dried pellets are then coated with release-controlling membrane (d) and filled into a two-piece, hard gelatin capsule.

| | mg/capsule |
|---|---|
| (a) Active Ingredient | 250 |
| (b) Microcrystalline Cellulose | 125 |
| (c) Lactose BP | 125 |
| (d) Ethyl Cellulose | 13 |
| | 513 |

Example 3: Injectable Formulation

| Formulation A. | |
|---|---|
| Active ingredient | 0.200g |
| Hydrochloric acid solution, 0.1M | q.s. to pH 4.0 to 7.0 |
| Sodium hydroxide solution, 0.1M | q.s. to pH 4.0 to 7.0 |
| Sterile water | q.s. to 10ml |

The active ingredient is dissolved in most of the water (35°-40°C) and the pH adjusted to between 4.0 and 7.0 with the hydrochloric acid or the sodium hydroxide as appropriate. The batch was then made up to volume with the water and filtered through a sterile micropore filter into a sterile 10ml amber glass vial (type 1) and sealed with sterile closures and overseals.

| Formulation B | |
|---|---|
| Active ingredient | 0.125 g |
| Sterile, pyrogen-free, pH 7 phosphate buffer, | q.s. to 25 ml |

Example 4: Intramuscular injection

| | Weight (g) |
|---|---|
| Active Ingredient | 0.20 |
| Benzyl Alcohol | 0.10 |
| Glycofurol 75 | 1.45 |
| Water for Injection | q.s. to 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example 5: Syrup

| Formulation A | |
|---|---|
| | Weight(g) |
| Active ingredient | 0.2500 |
| Sorbitol Solution | 1.5000 |
| Glycerol | 2.0000 |
| Sodium Benzoate | 0.0050 |
| Flavour, Peach | 0.0125 ml |
| Purified Water | q.s. to 5.0000 ml |

The active ingredient is dissolved in a mixture of the glycerol and most of the purified water. An aqueous solution of the sodium benzoate is then added to the solution, followed by addition of the sorbitol solution and finally the flavour. The volume is made up with purified water and mixed well.

When the active ingredient is poorly soluble, the following formulation (B) is used.

| Formulation B | |
|---|---|
| | Weight(g) |
| Active Ingredient | 0.250 |
| Sorbitol Solution | 1.500 |
| Glycerol | 0.005 |
| Dispersible Cellulose | 0.005 |
| Sodium Benzoate | 0.010 ml |
| Flavour | q.s. |
| Purified Water | q.s. to 5.000 ml |

Mix the sorbitol solution, glycerol and part of the purified water. Dissolve the sodium benzoate in purified water and add the solution to the bulk. Add and disperse the dispersible cellulose and flavour. Add and disperse the active ingredient. Make up to volume with purified water.

Example 6: Suppository

| | mg/suppository |
|---|---|
| Active Ingredient (63µm)* | 250 |
| Hard Fat, BP (Witepsol H15 - Dynamit NoBel) | 1770 |
| | 2020 |

*The active ingredient is used as a powder wherein at least 90% of the particles are of 63µm diameter or less.

One-fifth of the Witepsol H15 is melted in a steam-jacketed pan at 45°C maximum. The active ingredient is sifted through a 200µm sieve and added to the molten base with mixing, using a silverson fitted with a cutting head, until a smooth dispersion is achieved. Maintaining the mixture at 45°C, the remaining Witepsol H15 is added to the suspension and stired to ensure a homogenous mix. The entire suspension is passed through a 250µm stainless steel screen and, with continuous stirring, is allowed to cool to 40°C. At a temperature of 38°C to 40°C, 2.02g of the mixture is filled into suitable, 2 ml plastic moulds. The suppositories are allowed to cool to room temperature.

Example 7: Pessaries

| | mg/pessary |
|---|---|
| Active ingredient (63µm) | 250 |
| Anhydrous Dextrose | 380 |
| Potato Starch | 363 |
| Magnesium Stearate | 7 |
| | 1000 |

The above ingredients are mixed directly and pessaries prepared by direct compression of the resulting mixture.

Veterinary Formulations (Examples 8-11)

Example 8: Tablet for small animal use

| | Per tablet |
|---|---|
| Active ingredient | 120 mg |
| Maize Starch | 20.0 mg |
| Microcrystalline Cellulose | 100.0 mg |
| Magnesium Stearate | 1.5 mg |

The active ingredient, microcrystalline cellulose and maize starch are mixed together. Sufficient starch solution is added with continued mixing to produce a moist mass, which is passed through a sieve to produce granules. The magnesium stearate is sifted on. Tablets are produced by direct compression of the granules.

Example 9: Granules for In-Feed Medication

| | Weight(g) |
|---|---|
| Active ingredient | 6.0 |
| Povidone | 1.0 |
| Lactose | 93.0 |
| Aqueous alcohol mixture | qs. |

The active ingredient is mixed with the lactose. To this is added the aqueous alcohol containing the dissolved povidone. Sufficient aqueous alcohol is added to produce a moist mass, which is passed through a sieve to produce granules which are subsequently dried.

Example 10: Oral Paste

| | Weight(g) |
|---|---|
| Active ingredient | 24 |
| Xanthan Gum | 0.5 |
| Methyl Hydroxybenzoate | 0.1 |
| Polysorbate 80 | 0.1 |
| Purified Water | to 100.0 ml |

The polysorbate 80 and methyl hydroxybenzoate are dissolved in the bulk of the water. The xanthan gum is added and dispersed and allowed to swell. The active ingredient is added and dispersed and diluted to volume.

Example 11: Ointment

| | Weight(g) |
|---|---|
| Active ingredient | 12 |
| White Soft Paraffin | 88.0 |

The white soft paraffin is melted at 60°C. The active ingredient is added and dispersed, allowed to cool, and filled into collapsible metal tubes.

Example 12 Anti-Giardial Activity

Giardia lamblia WB strain (ATCC 30957) was grown in TYI-S-33 medium [D.B. Kiester, Transactions of the Royal Society of Tropical Medicine and Hygiene, Vol. 27, No. 4, p. 487-488 (1983)]. The compound of formula (I) was added to portions of the logarithmically growing culture to give concentraions of the compound ranging from about 1 µM to 17µM. The $ED_{50}$ values were determined after incubation at 37°C for 45.5 and 69.5 hr to be 7 µM at both time periods.

## Claims

**1.** Use of 3'-azido-3'-deoxythymidine, i.e. of formula (I):-

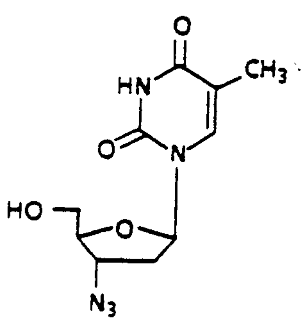

(I)

or a pharmaceutical acceptable salt and ester thereof in the manufacture of a pharmaceutical composition for the treatment or prophylaxis of a giardial infection in an animal.

**2.** Use of the compound of formula (I) or a, pharmaceutically acceptable salt or ester thereof according to claim 1 wherein the said animal is a human.

**3.** Use of the compound of formula (I) or a pharmaceutically acceptable salt or ester thereof according to claim 1 wherein the said pharmaceutical composition is adapted for oral, rectal or parenteral administration.

**4.** Use of the compound of formula (I) or a pharmaceutically acceptable salt or ester thereof according to claim 1 wherein the said pharmaceutical composition is in the form of tablets or capsules.

**5.** Use of the compound of formula (I) or a pharmaceutically acceptable salt or ester thereof according to claim 1 wherein the said pharmaceutical composition is employed in said treatment or prophylaxis in an amount of 1 to 100mg of compound of formula (I) per kilogram of body weight of the animal, per day.

**6.** Use of the compound of formula (I) or a pharmaceutically acceptable salt or ester thereof according to claim 1 wherein the said pharmaceutical composition is employed in said treatment or prophylaxis in an amount of 3 to 40mg of compound of formula (I) per kilogram of body weight of the animal per day.

## Revendications

1. Utilisation de la 3'-azido-3'-désoxythymidine, c'est-à-dire du composé de formule (I) :

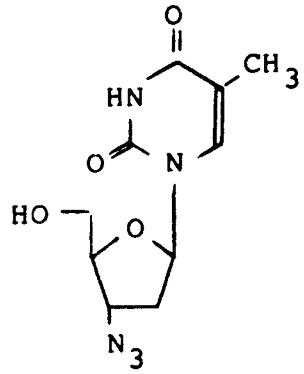

(I)

ou d'un sel ou ester pharmaceutiquement acceptable de celui-ci dans la fabrication d'une composition pharmaceutique pour le traitement ou la prophylaxie d'une infestation par Giardia chez un animal.

2. Utilisation du composé de formule (I) ou d'un sel ou ester pharmaceutiquement acceptable de celui-ci suivant la revendication 1, dans laquelle l'animal est un être humain.

3. Utilisation du composé de formule (I) ou d'un sel ou ester pharmaceutiquement acceptable de celui-ci suivant la revendication 1, dans laquelle la composition pharmaceutique est adaptée à l'administration par voie orale, rectale ou parentérale.

4. Utilisation du composé de formule (I) ou d'un sel ou ester pharmaceutiquement acceptable de celui-ci suivant la revendication 1, dans laquelle la composition pharmaceutique est présentée sous la forme de comprimés ou de capsules.

5. Utilisation du composé de formule (I) ou d'un d'un sel ou ester pharmaceutiquement acceptable de celui-ci suivant la revendication 1, dans laquelle la composition pharmaceutique est utilisée pour le traitement ou la prophylaxie en une quantité de 1 à 100 mg du composé de formule (I) par kg de poids du corps de l'animal et par jour.

6. Utilisation du composé de formule (I) ou d'un d'un sel ou ester pharmaceutiquement acceptable de celui-ci suivant la revendication 1, dans laquelle la composition pharmaceutique est utilisée pour le traitement ou la prophylaxie en une quantité de 3 à 40 mg du composé de formule (I) par kg de poids du corps de l'animal et par jour.

**Patentansprüche**

1. Verwendung von 3'-Azido-3'-deoxythymidin, d.h. mit der Formel (I)

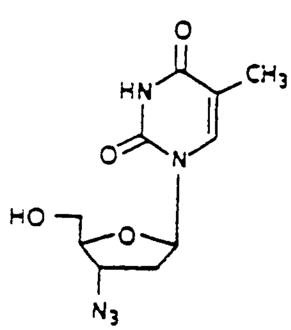

(I)

oder eines pharmazeutisch akzeptablen Salzes und eines Esters davon für die Herstellung einer

pharmazeutischen Zusammensetzung für die Behandlung oder Prophylaxe einer Giardiainfektion bei einem Lebewesen.

**2.** Verwendung der Verbindung gemäss Formel (I) oder eines pharmazeutisch akzeptablen Salzes oder Esters davon nach Anspruch 1, dadurch **gekennzeichnet,** dass das Lebewesen ein Mensch ist.

**3.** Verwendung der Verbindung gemäss Formel (I) oder eines pharmazeutisch akzeptablen Salzes oder Esters davon nach Anspruch 1, dadurch **gekennzeichnet,** dass die pharmazeutische Zusammensetzung für orale, rektale oder parenterale Verabreichung angepasst ist.

**4.** Verwendung der Verbindung (I) oder eines pharmazeutisch akzeptablen Salzes oder Esters davon nach Anspruch 1, dadurch **gekennzeichnet,** dass die pharmazeutische Zusammensetzung in der Form von Tabletten oder Kapseln vorliegt.

**5.** Verwendung der Verbindung gemäss Formel (I) oder eines pharmazeutisch akzeptablen Salzes oder Esters davon nach Anspruch 1, dadurch **gekennzeichnet,** dass die pharmazeutische Zusammensetzung bei der Behandlung oder Prophylaxe in einer Menge von 1 bis 100 mg der Verbindung der Formel (I) pro kg Körpergewicht des Lebewesens pro Tag verwendet wird.

**6.** Verwendung der Verbindung der Formel (I) oder eines pharmazeutisch akzeptablen Salzes oder Esters davon nach Anspruch 1, dadurch **gekennzeichnet,** dass die pharmazeutische Zusammensetzung bei der Behandlung oder Prophylaxe in einer Menge von 3 bis 40 mg der Verbindung der Formel (1) pro kg Körpergewicht des Lebewesens pro Tag verwendet wird.